(19) Europäisches Patentamt
European Patent Office
Office européen des brevets

(11) EP 1 490 326 B1

(12) # EUROPÄISCHE PATENTSCHRIFT

(45) Veröffentlichungstag und Bekanntmachung des Hinweises auf die Patenterteilung:
**29.12.2010 Patentblatt 2010/52**

(21) Anmeldenummer: **02758206.3**

(22) Anmeldetag: **28.05.2002**

(51) Int Cl.:
***C07C 213/06*** *(2006.01)*

(86) Internationale Anmeldenummer:
**PCT/EP2002/005818**

(87) Internationale Veröffentlichungsnummer:
**WO 2002/100817 (19.12.2002 Gazette 2002/51)**

(54) **VERFAHREN ZUR HERSTELLUNG VON (METH) ACRYLSAEUREESTERN**

METHOD FOR PRODUCING (METH)ACRYLIC ACID ESTERS

PROCEDE DE PRODUCTION D'ESTERS D'ACIDE (METH)ACRYLIQUE

(84) Benannte Vertragsstaaten:
**AT BE CH CY DE DK ES FI FR GB GR IE IT LI LU MC NL PT SE TR**

(30) Priorität: **08.06.2001 DE 10127938**

(43) Veröffentlichungstag der Anmeldung:
**29.12.2004 Patentblatt 2004/53**

(73) Patentinhaber: **BASF SE**
**67056 Ludwigshafen (DE)**

(72) Erfinder:
- **GEISENDÖRFER, Matthias**
  **67435 Neustadt (DE)**
- **NESTLER, Gerhard**
  **A-1070 Wien (AT)**
- **SCHRÖDER, Jürgen**
  **67071 Ludwigshafen (DE)**
- **VANDENMERSCH, Hugues**
  **67157 Wachenheim (DE)**

(56) Entgegenhaltungen:
**EP-A- 0 376 088**
**EP-A- 0 906 902**
**DE-A- 4 019 788**
**DE-A- 10 127 939**
**DE-B- 1 248 943**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung beschreibt ein Verfahren zur Herstellung von basischen (Meth)acrylsäureestern (IV) in hoher Reinheit und mit hoher Ausbeute durch Umesterung von technischem, niederen Alkyl(meth)acrylat I mit basischen Alkoholen ($R^2OH$).

Unter "hoher Reinheit" wird eine Reinheit von min. 99.8 % verstanden, wobei der Gesamtgehalt an gesättigten Verunreinigungen (ohne Wasser) nicht mehr als 1000 ppm und der Gehalt an N,N'-Dimethylpiperazin, Ethylenglykoldi(meth)acrylat und Vinyloxyethyl(meth)acrylat jeweils nicht mehr als 100 ppm beträgt.

(Meth)acrylsäureester sind wertvolle Ausgangsverbindungen zur Herstellung von Polymeren und Copolymeren, die z. B. als Lacke, Dispersionen oder Klebstoffe Anwendung finden.

Gesättigte Verunreinigungen, also solche, die keine Kohlenstoff-Kohlenstoff-Mehrfachbindungen aufweisen, wie z.B. Alkohole, Ether, Essig- und Propionsäurederivate, sind insofern nachteilig, da sie die Polymerisation unverändert überstehen, also nicht in das Polymerisat eingebaut werden, und zu einer Geruchsbelästigung im Produkt führen können. Um diese sogenannten "Restflüchtigen Bestandteile", beispielsweise aus Dispersionen abzutrennen, sind zusätzlich aufwendige Behandlungen (Desodorierungen) notwendig.

In der Regel wird eine als physikalische Desodorierung bezeichnete Behandlung durchgeführt, die darin besteht, daß die Dispersion mit Wasserdampf, Luft oder Stickstoff in einem Rührbehälter (DE-AS 12 48 943) oder in einer Gegenstromkolonne gestrippt wird. Abhängig von der Menge und den Siedepunkten der abzutrennenden Komponenten erfolgt die Behandlung ein- oder mehrstufig. Die Entfernung dieser Begleitstoffe ist demnach ein aufwendiger Vorgang, der außerdem bei temperaturempfindlichen Dispersionen wegen der thermischen Belastung nicht durchführbar ist.

Der Gehalt an Ether (Dibutylether wird beispielsweise mit Butylacrylat als niederem (Meth)acrylat I eingeschleppt; s. u.) wirkt sich zusätzlich negativ aus, wenn, z.B. zur Stabilisierung, in Gegenwart von sauerstoffhaltigen Gasen, wie z.B. Luft, gearbeitet wird. Ether bilden bekanntlich in Gegenwart von Sauerstoff sehr leicht Peroxide und diese können dann bekanntlich eine Polymerisation von (Meth)acrylverbindungen auslösen, die sogar explosionsartig erfolgen kann.

Der Ethergehalt stellt demnach nicht nur ein Qualitäts-, sondern auch ein Sicherheitsproblem dar.

Ethylenglykoldi(meth)acrylat und Vinyloxyethyl(meth)acrylat, die bei der Herstellung von Alkylaminoethyl(meth)acrylaten als Nebenkomponenten vorkommen, enthalten zwei ungesättigte Gruppen und wirken daher bei der Polymerisation als Vernetzer. Dies ist äußerst nachteilig, da dadurch die Polymerisation und die Qualität der Polymerisate, z.B. durch Gelbildung, beeinträchtigt wird. Außerdem beeinflussen sie die Lagerstabilität.

Die Herstellung von basischen (Meth)acrylaten IV durch Umesterung von niederen (Meth)acrylsäureestern I mit basischen Alkoholen $R^2OH$ ist allgemein bekannt.

Es ist weiterhin allgemein bekannt, daß die Umesterung eine Gleichgewichtsreaktion darstellt. Um wirtschaftliche Umsätze zu erzielen wird daher in der Regel das gebildete niedere Alkanol $R^1OH$ als Komponente mit dem niedrigsten Siedepunkt kontinuierlich destillativ aus dem Gleichgewicht entfernt, wobei aus wirtschaftlichen Gründen eine möglichst reine Alkanolfraktion angestrebt wird, um diese beispielsweise in der Herstellung des niederen (Meth)acrylsäureesters I durch Veresterung wieder einsetzen zu können. Aufgrund der Lage der Siedepunkte und der Ausbildung von Azeotropen besteht dieses Destillat aber in der Regel nicht aus reinem niederen Alkanol $R^1OH$, sondern ist mit dem niederen (Meth)acrylsäureester I sowie gegebenenfalls mit basischem Alkohol $R^2OH$ verunreinigt.

Da aus wirtschaftlichen Gründen eine Verwertung des Destillats sinnvoll ist, wirken sich Verunreinigungen negativ aus, insbesonders wenn es sich um basische Verunreinigungen, d.h. Verbindungen mit einer Aminogruppe, handelt.

Vor allem die besonders wirtschaftliche Rückführung in die Synthese des niederen Esters wird dadurch beeinflußt, siehe z.B. EP-A2 906 902, Seite 3, Zeilen 4 - 16.

EP-A2 906 902 beschreibt ein Verfahren zur Herstellung von Alkylamino(meth)acrylaten durch Umesterung von Alkyl(meth)acrylaten mit Alkylaminoalkoholen in Gegenwart eines Katalysators, z.B. Dibutylzinnoxid, in dem das alkoholhaltige Destillat (Azeotrop) entweder direkt oder nach einer weiteren Destillation über ein saures Ionenaustauscherharz geleitet wird. Die basischen stickstoffhaltigen Verunreinigungen aus dem Destillat werden durch die sauren Gruppen gebunden und dadurch aus dem Alkanol/(Meth)acrylester-Gemisch abgetrennt, das dann wieder bei der Synthese des niederen (Meth)acrylesters eingesetzt werden kann. Die Aufarbeitung des Umesterungsgemisches erfolgt in mehreren Destillationsstufen, wobei die zusätzliche Bildung der Michael-Additionsprodukte während der Katalysatorabtrennung möglichst reduziert wird.

Als Michael-Additionsprodukte werden die durch Addition von Alkoholen an die Doppelbindung der (Meth)acrylester entstandenen Verbindungen bezeichnet.

Es ist allgemein bekannt, daß diese Addition (s. Gleichung I) besonders in Anwesenheit von alkalischen Katalysatoren erfolgt (Organikum, 17. Auflage, Seite 506, VEB Deutscher Verlag der Wissenschaften, Berlin 1988).

Gleichung I

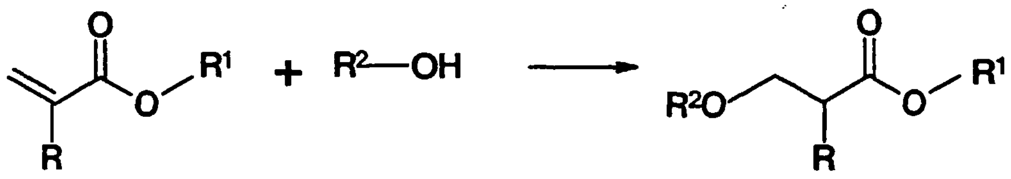

(I)

Durch eine "zweistufige Katalysatorabtrennung" (EP-A2 906 902, Seite 4, Zeilen 51 - 57) wird die zusätzliche Bildung ("ratio of increase") der Michael-Addukte unter 2 % gehalten. Entsprechend den Beispielen III-1, III-2 und III-3 spielen die Destillationstemperaturen und die Verweilzeiten eine entscheidende Rolle. Bei Erhöhung der Temperatur in der Stufe 2 beziehungsweise der Verweilzeit in beiden Stufen steigt nämlich die "ratio of increase" der Michael-Addukte deutlich an (von 0,48 auf 0,96 bzw. 2,2 %).

Der tatsächliche, absolute Gehalt der Michael-Addukte im Reaktionsgemisch, der für die Ausbeute und die Wirtschaftlichkeit des Verfahrens entscheidend ist, wird in EP-A 906 902 nirgends erwähnt.

Das Verfahren hat folgende Nachteile:

1. Notwendigkeit der Reinigung mit einem Ionenaustauscher
2. Die Regenerierung und Entsorgung des mit den basischen Verunreinigungen beladenen Austauscherharzes ist aufwendig und umweltbelastend.
3. Es benötigt 5 bis 7 Destillationsschritte und ist somit technisch aufwendig.
4. Die Ausbeute ist gering (ca. 33 %, Bsp. III-1).
5. Der Aminoalkohol muß über einen langen Zeitraum kontinuierlich zudosiert werden (4 Stunden, s. Bsp. III-1), um die Bildung der Michael-Produkte zu verringern.
6. Es werden lange Reaktionszeiten benötigt (7-8 Stunden), was die Wirtschaftlichkeit senkt.

Eigene Untersuchungen haben gezeigt, daß vor allem die Reaktionszeit (also die Verweilzeit in den Reaktoren) einen entscheidenden Einfluß auf die Bildung der Michael-Addukte hat (siehe Beispiel 3). Die Temperatur und die Verweilzeit bei der Katalysatorabtrennung haben dagegen überraschenderweise bei dem erfindungsgemäßen Verfahren für die Bildung der Michael-Addukte keine Bedeutung (s. Vergleichsbeispiele 1 und 2).

In der Regel werden bei der Umesterung (Meth)acrylsäuremethyl- und -ethylester als Ausgangsprodukte eingesetzt (EP-A 960 877, FR 2 617 840), Butyl(meth)acrylat wird wegen seines hohen Siedepunktes als nachteilig angesehen (US 2 832 800, Spalte 2, Zeilen 60-70).

Da in der Literatur zur Umesterung von Alkyl(meth)acrylaten keine näheren Angaben über die Begleitstoffe und Verunreinigungen in den eingesetzten Ausgangsestern gemacht werden, muß davon ausgegangen werden, daß die Reinheit der eingesetzten (Meth)acrylate sehr hoch ist und keine störenden Komponenten enthalten sind.

Die Verwendung von Estern hoher Reinheit ist jedoch nachteilig, da diese nach ihrer Herstellung technisch aufwendig destillativ gereinigt werden müssen. In Hinblick auf die allgemein bekannte hohe Polymerisationsneigung von (Meth)acrylverbindungen bei thermischer Belastung ist dies besonders nachteilig.

Als Katalysatoren für die Herstellung von (Meth)acrylsäureestern durch Umesterung werden vor allem Titanalkoholate vorgeschlagen, deren Alkylgruppen $C_1$ - $C_4$ - Alkylreste darstellen, z.B. Tetramethyl-, Tetraethyl-, Tetraisopropyl-, Tetrapropyl, Tetraisobutyl- und Tetrabutyltitanat (siehe z.B. EP-B1 298 867, EP-A2 960 877). Weiterhin werden als Katalysatoren u. a. Titanphenolate (DE-OS 200 86 18), Metallchelatverbindungen von z. B. Hafnium, Titan, Zirkon oder Calcium, Alkali- und Magnesiumalkoholate, organische Zinnverbindungen oder Calcium- und Lithiumverbindungen, beispielsweise -Oxide, -Hydroxyde, -Carbonate oder -Halogenide vorgeschlagen.

Aus ökonomischen und ökologischen Gründen werden vor allem Alkyltitanate eingesetzt, obwohl sie beispielsweise schon gegen Spuren von Wasser empfindlich und manche Titanalkoholate bei höheren Temperaturen instabil sind. Die Folge ist eine Belagsbildung (fouling, s.u.) an den Apparatewänden.

Weiterhin ist allgemein bekannt, daß Alkyltitanate die Polymerisation von (Meth)acrylestern fördern und daher zur Bildung von Polymerisat bei der Umesterung und der Aufarbeitung des Umesterungsgemisches Anlaß geben können (DE-OS 20 08 618, Seite 3, DE-PS 10 67 806, Spalte 1, Zeilen 39 - 41).

Nachteilig ist auch, daß Titanalkoholate wegen ihrer z. T. relativ geringen Aktivität erhöhte Umesterungstemperaturen notwendig machen, um wirtschaftliche Umsätze bzw. Reaktionszeiten zu erreichen (EP-A 160 427, Seite 2, Zeilen 23 - 32). Dies kann wiederum zu einer vermehrten Polymerisat- und Belagsbildung führen.

Ein Problem stellt auch der Aktivitätsverlust dar, den Titanalkoholate mit der Zeit erleiden (DE-OS 28 05 702, Seite 5, Zeilen 12 - 21). Um wirtschaftliche Umsätze zu erzielen, muß die Katalysatormenge erhöht und/oder die Reaktionszeit erhöht werden. In Hinblick auf die Instabilität der Titanate und die Nebenprodukt- und Polymerisatbildung ist dies bekanntlich nachteilig.

Hinzu kommt noch die Tatsache, daß (Meth)acrylsäureverbindungen eine große Neigung zur Polymerisation besitzen, ganz besonders, wenn Hitze auf sie einwirkt. Vor allem bei der Herstellung und der destillativen Reinigung sind sie Temperaturen ausgesetzt, die leicht eine unerwünschte Polymerisation auslösen können. Auch der Einsatz von Polymerisationsinhibitoren, wie er allgemein empfohlen wird, kann die Polymerisatbildung nicht vollständig verhindern.

Verschmutzung der Apparaturen, Verstopfen von Leitungen und Pumpen und die Belegung von Kolonnenböden und Wärmetauscherflächen ("fouling") sind in der Regel die Folge einer Polymerisatbildung. Das Reinigen der Anlagen ist ein aufwendiger, teurer und umweltbelastender Vorgang, die Ausbeute und die Verfügbarkeit der Anlagen (Laufzeit) wird dadurch außerdem stark reduziert.

JP-A 3-112 949 beschreibt ein Verfahren zur Herstellung von Dimethylaminoethylacrylat durch Umesterung von n-Butylacrylat mit Dimethylaminoethanol unter Verwendung von Tetra-n-butyltitanat, bei dem die Reinigung des Reaktionsgemisches in Abwesenheit von Sauerstoff erfolgt.

Nachteilig bei diesem Verfahren sind jedoch die Ausbeuten, die unter 90 % liegen.

Es wurde nun gefunden, daß die vorgenannten Probleme in einem Verfahren zur Herstellung von basischen (Meth)acrylsäureestern IV durch Umesterung von (Meth)acrylsäurealkylestern I in Gegenwart eines Katalysators und destillativer Aufarbeitung des Reaktionsgemisches verringert werden, wenn man ein unter den Reaktionsbedingungen inertes Gas oder Gasgemisch durch die Reaktionszone und/oder Wärmetauscher leitet.

Die Umsetzung läßt sich durch Reaktionsgleichung II wiedergeben:

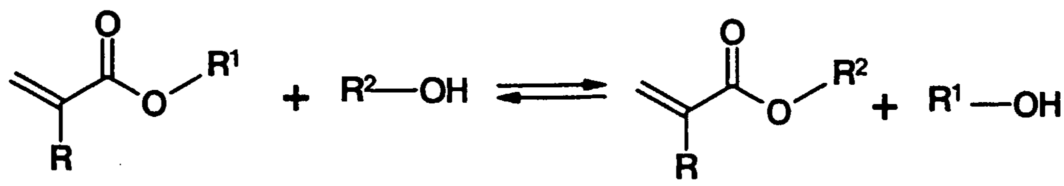

(I) (IV)

Darin ist

- R Wasserstoff oder Methyl,
- $R^1$ ein Alkylrest eines ein bis sechs Kohlenstoffatome auf- weisenden Alkohols,
- $R^2$ ein zwei bis zwölf Kohlenstoffatome umfassender, gerad- kettiger oder verzweigter, gesättigter oder ungesättigter Alkylrest, substituiert mit mindestens einer $NR^3_2$ - Gruppe
- $R^3$ ein zwei bis sechs Kohlenstoffatome umfassender, gerad- kettiger oder verzweigter, gesättigter oder ungesättigter Alkylrest, wobei N mit den Substituenten $R^3$ auch einen fünf- bis siebengliedrigen Ring bilden kann und die Substituenten $R^3$ gleich oder verschieden sein können.

$R^1$ soll dabei mindestens ein Kohlenstoffatom weniger enthalten als $R^2$.

Mit niederem Alkyl(meth)acrylat I sind dabei beispielsweise (Meth)acrylsäureester von ein bis sechs Kohlenstoffatome aufweisenden Alkoholen bezeichnet, z.B. von Methanol, Ethanol, *iso*-Propanol, n-Propanol, n-Butanol, *iso*-Butanol, *sek*-Butanol, tert-Butanol, n-Pentanol oder n-Hexanol, bevorzugt die von Methanol, Ethanol und n-Butanol, besonders bevorzugt die von n-Butanol. Insbesondere bevorzugt ist n-Butylacrylat.

Bevorzugte basische Alkohole $R^2OH$ sind 2-Dimethylaminoethan-1-ol, 3-Dimethylaminopropan-1-ol, 1-Dimethylaminopropan-2-ol, 2-Dimethylaminopropan-1-ol, 6-Dimethylaminohexan-1-ol, 2-Diethylaminoethan-1-ol, 3-Diethylaminopropan-1-ol, 6-Diethylaminohexan-1-ol, 2-Dibutylaminoethan-1-ol, 3-Dibutylaminopropan-1-ol und 6-Dibutylaminohexan-1-ol, besonders bevorzugt sind die genannten Dialkylaminoethanole, insbesondere bevorzugt ist 2-Dimethylaminoethan-1-ol.

Unter "hoher Reinheit" wird hier eine Reinheit von min. 99.8 % verstanden, wobei der Gesamtgehalt an gesättigten Verunreinigungen (ohne Wasser) nicht mehr als 1000 ppm, bevorzugt bis zu 500 ppm und an N,N'-Dimethylpiperazin, Ethylenglykoldi(meth)acrylat und Vinyloxyethyl(meth)acrylat jeweils nicht mehr als 100, bevorzugt nicht mehr als 50 und besonders bevorzugt nicht mehr als 20 ppm und insbesondere nicht mehr als 10 ppm beträgt.

Unter technischem Alkylacrylat und technischem Alkylmethacrylat werden die im industriellen Maßstab hergestellten niederen (Meth)acrylsäureester I verstanden, die in der Regel eine Reinheit von 99,0 - 99,8 % aufweisen. Als Verunreinigungen enthalten diese (Meth)acrylate im wesentlichen Dialkylether (0,01 - 0,2 %), Alkylacetat (0,01 - 0,1 %), Alkylpropionat (0,02 - 0,1 %), Alkanol $R^1OH$ (0,01 - 0,05 %), Wasser (0,01 - 0,05 %), (Meth)acrylsäure (0,001 - 0,1 %) und andere, z.B. isomere Alkylacrylate (0,01 - 0,3 %).

Das erfindungsgemäße Verfahren wird im folgenden exemplarisch für Acrylsäure-n-butylester als I beschrieben, ohne es darauf zu begrenzen, kann aber in analoger Weise auch auf andere niedere Alkohole $R^1OH$ als n-Butanol übertragen werden, beispielsweise $C_1$ bis $C_6$-Alkohole, z.B. Methanol, Ethanol, *iso*-Propanol, n-Propanol, *iso*-Butanol oder n-Pentanol. Dazu wird statt n-Butanol der betreffende Alkohol beziehungsweise der betreffende niedere (Meth) acrylsäureester I eingesetzt, wobei die Betriebsparameter den veränderten Siedepunkten und sonstigen physikalischen Eigenschaften der Edukte und Produkte angepaßt werden, was im Rahmen fachüblicher Versuche möglich ist.

Das erfindungsgemäße Verfahren kann analog prinzipiell auch auf Methacrylsäureester angewendet werden.

Der niedere (Meth)acrylsäureester I wird im erfindungsgemäßen Verfahren in technischer Reinheit, wie eingangs erwähnt, eingesetzt.

Der in die Umesterung eingesetzte niedere (Meth)acrylsäureester I hat in technischer Reinheit in der Regel eine Zusammensetzung wie oben aufgeführt.

Selbstverständlich kann auch niederer (Meth)acrylsäureester I mit einer höheren Reinheit, z.B. bis zu 99,95 Gew%, eingesetzt werden, wobei dann die anderen Verunreinigungen in entsprechend geringerem Anteil enthalten sind.

Der höhere Alkohol $R^2OH$ hat üblicherweise eine Reinheit von min. 99,0 Gew% und einen Wassergehalt von 0,01 - 0,2 Gew%.

Der Gehalt an Ethylenglykol im Fall von Dialkylaminethanolen als höherem Alkohol $R^2OH$ sollte nicht mehr als 100 ppm betragen, bevorzugt nicht mehr als 50 ppm, besonders bevorzugt nicht mehr als 20 ppm und insbesondere nicht mehr als 10 ppm. Der Gehalt an Vinyloxyethanol im höheren Alkohol $R^2OH$ sollte nicht mehr als 100 ppm betragen, bevorzugt nicht mehr als 50 ppm, besonders bevorzugt nicht mehr als 20 ppm und insbesondere nicht mehr als 10 ppm.

Im Fall von Dialkylaminoethanolen können auch Spuren, beispielsweise bis zu 200 ppm, bevorzugt weniger als 100 ppm, an höheren Homologen enthalten sein.

Der Gehalt an N,N'-Dimethylpiperazin beträgt in der Regel nicht mehr als 100 ppm, bevorzugt nicht mehr als 50 ppm, besonders bevorzugt nicht mehr als 20 ppm und insbesondere nicht mehr als 10 ppm.

Es können auch Gemische von höheren Alkoholen zur Umesterung verwendet werden.

Die Umesterung kann auf an sich bekannte Weise, beispielsweise wie folgt durchgeführt werden: Der niedere (Meth)acrylsäureester I, vorzugsweise der Methyl-, Ethyl- oder n-Butylester, besonders bevorzugt der n-Butylester, wird mit dem höheren Alkohol $R^2OH$ in einem molaren Verhältnis von Ester : Alkohol 1 : 1 - 4 : 1 in Gegenwart mindestens eines Katalysators umgesetzt.

Die Umesterung kann diskontinuierlich, halbkontinuierlich oder kontinuierlich durchgeführt werden, bevorzugt kontinuierlich.

Für das erfindungsgemäße Verfahren können sämtliche im Stand der Technik beschriebenen Umesterungskatalysatoren eingesetzt werden, vorzugsweise Titan-, Magnesium- oder Aluminiumalkoholate, besonders bevorzugt Titanalkoholate und insbesondere Titanalkoholate der Alkohole, die in der Umesterung vorliegen, also $R^1OH$ und $R^2OH$.

Wenn als Titankatalysator ein niederes Alkoholat eingesetzt wird, das nicht die Alkoholkomponente $R^1OH$ enthält, wird in einer bevorzugten Ausführungsform der Katalysator vorab hergestellt wie folgt:

Ein niederes Titanalkoholat $Ti(OR^4)_4$, vorzugsweise das Isopropylat, Isobutylat bzw. n-Butylat, wird mit dem höheren Alkohol $R^2OH$ (siehe Gleichung III) bei erhöhter Temperatur (50 - 130 °C) zur Reaktion gebracht. Dabei wird der höhere Alkohol $R^2OH$ in molarem Überschuß (in der Regel 1:5 bis 1:20) eingesetzt.

Gleichung III

$$Ti(OR^4)_4 + R^2OH \rightleftharpoons Ti(OR^2)_4 + R^4OH$$

$R^2$ s. Gleichung II

$R^4$ steht für $C_1$ - $C_8$ - Alkyl, vorzugsweise für Isopropyl, Iso- butyl- oder n-Butyl

$R^2OH$ und $R^4OH$ sollen dabei über ihre Siedepunkte Kp vorzugsweise folgende Bedingung erfüllen:

$$Kp. (R^2OH) \geq Kp. (R^4OH) + 20\ °C$$

Unter diesen Bedingungen ist es technisch einfach, die Verluste an $R^2OH$ gering zu halten und $R^4OH$ möglichst vollständig abzutrennen.

Der bei der Reaktion entstehende Alkohol $R^4OH$ wird, gegebenenfalls bei vermindertem Druck, destillativ oder rektifikativ abgetrennt. Dies kann gegebenenfalls durch Strippen mit einem geeigneten, reaktionsträgen Gas unterstützt werden. Der anfallende Rückstand stellt die Katalysatorlösung für die Umesterung dar (Ti - Gehalt: 2 - 10 Gew%) und enthält in der Regel weniger als 400 ppm $R^4OH$. Es wird somit praktisch kein Fremdalkohol ($R^4OH$) in das Umesterungsgemisch (< 100 ppm im Gemisch) eingeschleust.

Selbstverständlich können in der Katalysatorlösung jedoch auch gemischte Titanalkoholate enthalten sein, in Abhängigkeit der Umsetzung nach Gleichung III.

Bei Verwendung eines Titanats, z.B. eines nach der oben angeführten Vorschrift hergestellten, beträgt der Titangehalt im Reaktionsgemisch in der Regel 0,01 - 1 Gew%.

Die Umesterung erfolgt in einem oder mehreren in Serie geschaltenen Reaktor(en) mit mindestens einer aufgesetzten Rektifikationskolonne und Kondensatoren.

Die Reaktionstemperatur beträgt in der Regel 80 - 140°C, bevorzugt 100 bis 130 °C, der Druck 200 mbar bis Atmosphärendruck, bevorzugt 300 - 800 mbar und besonders bevorzugt 400 bis 600 mbar.

Bei mehreren Reaktoren kann die Temperatur in den unterschiedlichen Reaktoren gleich oder unterschiedlich sein, z.B. im Verlauf der Reaktoren steigen oder fallen, bevorzugt steigt sie an.

Die Wärmezufuhr kann über eine Wandbeheizung oder/und außen- oder innenliegende Wärmetauscher, z. B. Röhren- oder Plattenwärmetauscher, vorzugsweise über außenliegende Umlaufverdampfer erfolgen. Die Rektifikationskolonnen sind von bekannter Bauart und haben trennwirksame Einbauten (z. B. Glocken-, Thormann-, Ventil-, Sieb- oder Dual-Flow-Böden) oder enthalten Schüttungen oder gerichtete Packungen. Die Kondensatoren sind ebenfalls von bekannter Bauart und können indirekt, z. B. als Röhren- oder Plattenwärmetauscher, oder direkt, z.B. als Quenchkühler, betrieben werden. Die gleichmäßige Durchmischung der Reaktionslösung erfolgt auf bekannte Weise, z.B. durch Rühren, Umpumpen, Zwangs- oder Naturumlauf, vorzugsweise durch Zwangs- oder Naturumlauf.

Die Reaktionszone und/oder die in der Anlage eingebauten Wärmetauscher, z.B. der Destillationseinheiten oder Reaktoren, wird/werden erfindungsgemäß mit einem unter den Reaktionsbedingungen inerten Gas oder Gasgemisch, z.B. Stickstoff, Luft, Stickstoff - Sauerstoff - Gemische, Argon, Helium, Kohlenstoffdi- oder -monooxid, vorzugsweise Luft oder Luft-Stickstoffgemische, insbesondere solche mit einem Sauerstoffgehalt von 0,1 bis zu 15 Vol%, bevorzugt von 0,5 bis zu 10 Vol% und ganz besonders bevorzugt solche Luft-Stickstoffgemische mit einem Sauerstoffgehalt von 1 bis 5 Vol% kontinuierlich gespült. Bevorzugt wird das Spülgas durch das Reaktionsgemisch oder entlang der vorhandenen Wärmetauscherflächen geleitet, besonders bevorzugt in einem vorhandenen Zwangs- oder Naturumlaufverdampfer.

Dazu wird das Spülgas druck- oder volumengeregelt durch eine geeignete, an sich bekannte, nicht beschränkte Zuführvorrichtung in der Nähe der vorhandenen Wärmetauscherfläche eindosiert, so daß der, bevorzugt kontinuierliche, Spülgasstrom im Gegen- oder Gleichstrom zur Flüssigkeit entlang der Wärmetauscherfläche geführt wird.

Das Spülgas kann auf die Temperatur des Wärmetauschermediums vortemperiert werden, so daß die Temperatur des Spülgases beispielsweise nicht mehr als 15 °C von der Temperatur des Wärmemediums differiert, bevorzugt nicht mehr als 10 °C.

Pro Stunde werden, bezogen auf das Volumen des Reaktionsgemisches (= 1 Volumenteil) in den Reaktoren und Nachreaktoren in der Reaktionszone, jeweils 0,1 - 100 Volumenteile Spülgas durch Wärmetauscher beziehungsweise die Reaktionszone geführt, bevorzugt 0,2 - 80 Volumenteile, besonders bevorzugt 0,5 - 70 Volumenteile und insbesondere 1 - 50 Volumenteile.

In besonders bevorzugter Weise wird das Spülgas über die Wärmetauscher geleitet, in denen das Reaktionsmedium in den Reaktoren beziehungsweise in den Destillationskolonnen erhitzt wird.

Eine besondere Ausführungsform der erfindungsgemäßen Umesterung besteht darin, daß die Reaktion in mindestens einem Reaktor mit aufgesetzter Kolonne durchgeführt wird und das Reaktionsgemisch kontinuierlich in einen Nachreaktor geführt wird, der gasseitig mit einem, bevorzugt dem letzten Umesterungsreaktor oder der aufgesetzten Kolonne verbunden ist.

Die Temperatur im Nachreaktor liegt in der Regel 1 - 10 °C höher als im Reaktor.

Die Verweilzeit in der Reaktionszone, umfassend den/die Reaktor(en) und gegebenenfalls den/die Nachreaktor (en) beträgt 1 - 4, vorzugsweise 1,5 - 3 Stunden.

Die dem/den Reaktor(en) aufgesetzte(n) Kolonne(n) hat/haben in der Regel 10 - 30 theoretischen Böden. Das Rücklaufverhältnis liegt in der Regel bei 5 - 20 : 1, vorzugsweise bei 7 - 15 : 1. Die Bedingungen dieser Destillation werden allgemein so gewählt, daß die Butanolfraktion am Kopf der dem Reaktor aufgesetzten Kolonne 5 - 30 %, vorzugsweise 10 - 20 % n-Butyl-(meth)acrylat enthält. In der Regel sind nicht mehr als 1, bevorzugt nicht mehr als 0,5 und besonders bevorzugt nicht mehr als 0,3 Gew% des höheren Alkohols $R^2OH$ enthalten.

Der bei der Umesterung freigesetzte niedere Alkohol $R^1OH$ wird gemeinsam mit einem Teil des niederen (Meth) acrylsäureesters I über den Kopf der den Reaktoren aufgesetzten Rektifikationskolonnen abgetrennt.

Die Destillationsbedingungen, z.B. die Trennstufen und das Rücklaufverhältnis, werden dabei vorzugsweise so gewählt, daß am Kopf der Kolonne ein nichtazeotropes Gemisch abgenommen wird, bei dem gegenüber der azeotropen Zusammensetzung aus niederem Alkanol $R^1OH$ und niederem (Meth)acrylsäureester I bei den entsprechenden Bedingungen der Gehalt an niederem (Meth)acrylsäureester I erhöht ist.

Das Destillat kann direkt, d. h. ohne einen zusätzlichen Reinigungsschritt, in die Synthese von n-Butyl(meth) acrylat zurückgeführt werden, wo es mit (Meth)acrylsäure wieder zum Ausgangsester I umgesetzt werden kann, wie es in der deutschen Patentanmeldung mit dem Titel "Verfahren zur Herstellung von (Meth)acrylsäureestern", mit dem gleichen Anmeldetag wie die vorliegende Schrift und dem Aktenzeichen 101 27 941.8 beschrieben ist. Vorteilhaft kann es dort dem Aufarbeitungsprozeß zugeführt werden, besonders bevorzugt einem Extraktionsprozeß.

Die Stabilisierung der Kolonnen kann mit den gängigen Stabilisatoren oder Gemischen davon erfolgen, wie z.B. N-Oxyle, wie z.B. 4-Hydroxy-2,2,6,6-tetramethylpiperidin-N-Oxyl oder 4-Oxo-2,2,6,6-tetramethylpiperidin-N-Oxyl, Phenole und Naphthole, wie z.B. p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-Methyl-2,6-*tert*.-Butylphenol (2,6-*tert*.-Butyl-p-Kresol) oder 4-*tert*.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Weiterhin können dies auch Abbauprodukte oder Derivate von Stabilisatoren sein, beispielsweise das Michael-Addukt von (Meth)acrylsäure beziehungsweise (Meth)acrylsäureester und Hydrochinon.

Die Stabilisierung kann in An- oder Abwesenheit von molekularem Sauerstoff erfolgen, bevorzugt in dessen Anwesenheit.

Bevorzugt erfolgt die Stabilisierung mit Phenothiazin, Hydrochinon, Hydrochinonmonomethylether, 4-Hydroxy-2,2,6,6-tetramethylpiperidin-N-Oxyl, 4-Oxo-2,2,6,6-tetramethylpiperidin-N-Oxyl, 2,6-*tert*.-Butyl-p-Kresol oder Gemischen davon in Mengen von jeweils zwischen 10 und 5000 ppm, besonders bevorzugt wird Phenothiazin oder ein phenothiazinhaltiges Gemisch, insbesondere ein Phenothiazin/4-Hydroxy-2,2,6,6-tetramethylpiperidin-N-Oxyl-Gemisch für die Stabilisierung verwendet. Die Zugabe kann jeweils über die Ausgangsstoffe, direkt oder über die Rückführ- oder Rücklaufströme erfolgen.

Insbesondere erfolgt die Stabilisierung mit dem mit 100-1000 ppm Phenothiazin und 10 - 500 ppm 4-Hydroxy-2,2,6,6-tetramethylpiperidin-N-Oxyl versetzten Rücklauf.

Vorzugsweise erfolgt die Stabilisierung als Lösung dieses Stabilisatorgemisches im niederen (Meth)acrylsäureester.

In besonders bevorzugter Weise wird das gelöste Stabilisatorgemisch auf die Kondensatorflächen aufgesprüht.

Das gebildete Reaktionsgemisch wird, bevorzugt kontinuierlich, aus dem Reaktor ausgetragen und in einer Destillationseinheit (Katalysatorabtrennung) in ein Kopfprodukt, das hauptsächlich den Zielester und die Ausgangstoffe enthält, und ein Sumpfprodukt, das im wesentlichen Zielester, Katalysator, hochsiedende Nebenprodukte und Polymerisationsinhibitoren enthält, aufgetrennt. Das Sumpfprodukt kann zumindest teilweise, bevorzugt zu 50 bis 100%, in den ersten Reaktor zurückgeführt werden. Der eventuell vorhandene Rest kann beispielsweise einer Rückstandsbehandlung unterworfen werden.

Die Destillationseinheit kann beispielsweise aus einem herkömmlichen Zwangs- oder Naturumlaufverdampfer und einer Kolonne, z.B. mit 5 - 15 theoretische Böden, üblicher Bauart bestehen. Die Rektifikationskolonnen des erfindungsgemäßen Verfahrens sind von bekannter Bauart und weisen trennwirksame Einbauten (z. B. Glokken-, Thormann-, Ventil-, Sieb- oder Dual-Flow-Böden) auf oder enthalten Schüttungen oder gerichtete Packungen.

Auch die Verdampferflächen der Katalysatorabtrennung können erfindungsgemäß wie oben beschrieben mit einem Spülgas gespült werden.

Die Sumpftemperatur beträgt in der Regel 80 - 160, bevorzugt 90 - 150 und besonders bevorzugt 90 - 120 °C, der entsprechende Druck 20 - 500, bevorzugt 50 - 300 und besonders bevorzugt 80 - 150 mbar. Das Rücklaufverhältnis liegt in der Regel bei 5:1 - 1:5, bevorzugt 3:1 - 1:3 und besonders bevorzugt bei 2 : 1 bis 1 : 2.

Die Destillation kann auch bei höherer Temperatur vorgenommen werden, um eine Rückspaltung von Michael-Addukten zu bewirken, wie in der deutschen Patentanmeldung mit dem Titel "Verfahren zur Herstellung von (Meth) acrylsäureestern" mit dem gleichen Anmeldetag wie die vorliegende Schrift und dem Aktenzeichen 101 27 939.6 beschrieben ist.

Gegebenenfalls kann der Destillationsprozeß durch das Durchleiten eines unter den Reaktionsbedingungen im wesentlichen inerten Gasstromes wie oben beschrieben (Strippen), wie z.B. Stickstoff, aber auch ein sauerstoffhaltiges Gas, wie z.B. Luft oder Luft-Stickstoffgemische, insbesondere solche mit einem Sauerstoffgehalt von 0,1 bis zu 15 Vol%, bevorzugt von 0,5 bis zu 10 Vol% und ganz besonders bevorzugt solche Luft-Stickstoffgemische mit einem Sauerstoffgehalt von 1 bis 5 Vol%, unterstützt werden. Bevorzugt wird das Durchleiten des erfindungsgemäßen Spülgases mit

dem Strippprozeß verbunden.

Um die Polymerisatbildung in der Destillationeinheit zu verhindern, wird vorteilhaft eine ca. 0,1 bis 1 %ige Lösung Phenothiazin im Ausgangsester in die Kondensatoren eingesprüht. Bevorzugt erfolgt die Stabilisierung mit dem mit 100-1000 ppm Phenothiazin und 10 - 500 ppm 4-Hydroxy-2,2,6,6-tetramethylpiperidin-N-Oxyl versetzten Rücklauf.

In besonders bevorzugter Weise wird das gelöste Stabilisatorgemisch auf die Kondensatorflächen aufgesprüht.

Das den Zielester enthaltende Destillat der Katalysatorabtrennung kann in einer weiteren Destillationseinheit (Reindestillation) in eine Leichtsiederfraktion, die hauptsächlich aus den Ausgangstoffen besteht und in die Umesterung zurückgeführt werden kann, in eine Schwersiederfraktion (Sumpf), die hauptsächlich Zielester und Inhibitoren enthält und vorteilhaft in die erste Destillationseinheit zurückgeführt wird, aber auch teilweise in die Rückstandsbehandlung geleitet werden kann, und eine Mittelsiederfraktion, die im wesentlichen den Zielester enthält, aufgetrennt werden. Der Zielester wird, vorzugsweise gasförmig, über einen Seitenabzug im unteren Kolonnenbereich, bevorzugt in der unteren Hälfte, besonders bevorzugt im unteren Drittel, ausgeschleust und kondensiert. Der Zielester wird mit 10 - 20 ppm Hydrochinonmonomethylether oder 2,6-tert.-Butyl-p-Kresol stabilisiert, bevorzugt durch Aufsprühen auf den Kondensator im gasförmigen Seitenabzug.

Die Kolonne hat in der Regel 10 - 30 theoretische Böden.

Die Kondensatoren und Verdampfer sind wie oben beschrieben ebenfalls von bekannter Bauart, z. B. Röhren- oder Plattenwärmetauscher.

Das Erwärmen erfolgt auf ebenfalls bekannte Weise, vorzugsweise durch Natur- oder Zwangsumlauf.

Auch die Verdampferflächen der Reindestillation können erfindungsgemäß wie oben beschrieben mit einem Spülgas gespült werden.

Die Sumpftemperatur beträgt in der Regel 80 - 150, bevorzugt 80 - 140 und besonders bevorzugt 90 - 130 °C, der entsprechende Druck 20 - 500, bevorzugt 30 - 300 und besonders bevorzugt 40 - 200 mbar. Das Rücklaufverhältnis liegt bei 5:1 bis 1:15 und bevorzugt bei 2:1 bis 1:10.

Die nicht als Rücklauf verwendete Leichtsiederfraktion kann ganz oder teilweise in die Umesterung zurückgeführt werden, direkt in einen Reaktor oder über eine darauf aufgesetzte Kolonne.

Der Sumpf kann zu 50 bis 100% in die Katalysatorabtrennung zurückgeführt werden, bevorzugt zu 75 bis 100 und besonders bevorzugt zu 90 bis 100 %. Der Rest kann in die Rückstandsbehandlung geleitet werden.

Um die Polymerisatbildung in den Destillationeinheiten zu verhindern, wird vorteilhaft eine Lösung von ca. 0,5 % Phenothiazin und 0,05 % 4-Hydroxy-2,2,6,6-tetramethylpiperidin-N-Oxyl im Ausgangsester auf die Kondensatoren aufgesprüht. Bevorzugt erfolgt die Stabilisierung mit dem mit 100-1000 ppm Phenothiazin und 10 - 500 ppm 4-Hydroxy-2,2,6,6-tetramethylpiperidin-N-Oxyl versetzten Rücklauf.

Eine besondere Ausführungsform des Verfahrens besteht darin, daß der Sumpfablauf der Katalysatorabtrennung oder gegebenenfalls ein Teil davon sowie gegebenenfalls der Reindestillation einer kontinuierlich oder diskontinuierlich betriebenen thermischen Behandlung (Rückspaltung, Destillation) unterworfen wird. Diese Rückstandsbehandlung, in der noch vorhandener Zielester zurückgewonnen wird und gleichzeitig die Michael-Additionsprodukte in die entsprechenden Alkohole und (Meth)acrylsäureester zurückgespalten und abgetrennt werden, ist nicht beschränkt.

Die Rückstandsbehandlung kann kontinuierlich oder diskontinuierlich in mindestens einem Reaktor, gegebenenfalls mit aufgesetzter Kolonne, betrieben werden, bevorzugt kontinuierlich.

In der Rückstandsbehandlung werden beispielsweise Michael-Additionsprodukte und Polymere rückgespalten.

Diese Michael-Additionsprodukte sind im allgemeinen in ihre Ausgangsverbindungen rückspaltbar, z.B. durch thermische und/oder katalytische Behandlung, z.B. in Gegenwart eines geeigneten Katalysators.

Der der Rückstandsbehandlung zugeführte Strom kann, gegebenenfalls unter Zusatz von weiteren, z.B. basichen oder sauren, Katalysatoren, zur Rückspaltung der enthaltenen Michael-Additionsprodukte thermisch behandelt werden.

Die Temperatur in der Rückstandsbehandlung beträgt im allgemeinen 100 bis 220 °C, bevorzugt 120 bis 200 °C, besonders bevorzugt 140 bis 180 °C und insbesondere 150 bis 180 °C.

Die Abtrennung der Leichtsieder aus der Rückstandsbehandlung kann durch das Durchleiten eines unter den Reaktionsbedingungen im wesentlichen inerten Gasstromes (Strippen), wie z.B. Stickstoff aber auch ein sauerstoffhaltiges Gas, wie z.B. Luft oder Luft-Stickstoffgemische, unterstützt werden.

Auf diese Weise können im allgemeinen 50 % oder mehr der enthaltenen Wertprodukte (Zielester und Edukte) zurückgewonnen werden.

Die abgetrennten Wertprodukte können dann in den Reaktor der Umesterung, gegebenenfalls über eine aufgesetzte Kolonne, rückgeführt werden, vorzugsweise in die Katalysatorabtrennung oder Reindestillation.

Es sind daher keine speziellen, technisch aufwendigen Maßnahmen notwendig, um die Bildung der Michael-Additionsprodukte während der Synthese und/oder während der Aufarbeitung zu minimieren.

Der Rückstand der Rückstandsbehandlung kann dann ein weiteres Mal einer Rückstandsbehandlung oder einer geeigneten Entsorgung, z.B. einer Verbrennung zugeführt werden.

Anstelle einer Rückstandsbehandlung können die Sümpfe des Verfahrens selbstverständlich auch ohne Behandlung entsorgt werden.

Das nach dem beschriebenen Verfahren gewonnene basische (Meth)acrylat hat entsprechend der gaschromatographischen Analyse eine Reinheit von 99,9 % oder mehr.

Der Gehalt an gesättigten Nebenkomponenten ist in der Regel < 400 ppm, der von N,N'-Dimethylpiperazin, Ethylenglykoldi(meth)acrylat und Vinyloxyethyl(meth)acrylat < 100 ppm.

Vorteile des Verfahrens:

- Das Verfahren kann vollkontinuierlich betrieben werden und benötigt nicht mehr als 3 Destillationskolonnen, mit Rückstandsbehandlung vier. Die Investitionskosten und Wartungs-/Reparaturkosten sind daher relativ gering.
- Es kann technisches n-Butyl(meth)acrylat eingesetzt werden, ohne daß dabei Schwierigkeiten in der Anlage oder Qualitätsprobleme auftreten.
- Mit dem erfindungsgemäßen Verfahren wird eine hohe Ausbeute erzielt, d. h. geringe Rückstandsmengen und damit geringe Unweltbelastung und niedrige Herstellkosten.
- Hohe Reinheit, d. h. geringer Gehalt an Nebenprodukten und damit ausgezeichnete Weiterverarbeitungseigenschaften.

Die erfindungsgemäß hergestellten Dialkylaminoalkyl(meth)acrylate, besonders Dialkylaminoethyl(meth)acrylate und speziell Dimethylaminoethyl(meth)acrylate sind wertvolle Monomere für die Herstellung von Copolymerisaten. Als Monomere werden sie in der vorliegenden Form oder nach Quaternisierung in die Polymerisation eingesetzt.

Übliche Quaternisierungsmittel sind beispielsweise Benzylhalogenide wie z.B. Benzylchlorid, Alkylhalogenide wie z.B. Methylchlorid, Ethylchlorid, Methylbromid, Ethylendichlorid oder Allylchlorid, Alkylenoxide wie z.B. Ethylenoxid, Propylenoxid, Styroloxid, *iso*-Butylenoxid oder Vinyloxiran, bevorzugt Ethylenoxid oder Propylenoxid und besonders bevorzugt Ethylenoxid, Alkylphosphite oder -phosphonate wie z.B. Trimethylphosphit oder Triethylphosphit, Dialkylsulfate wie z.B. Dimethylsulfat oder Diethylsulfat, Dialkylcarbonate wie z.B. Dimethylcarbonat, Diethylcarbonat oder Di-n-butylcarbonat, Chlorhydrin oder Epichlorhydrin.

Besonders solche Copolymere, die quaternisierte Monomere einpolymerisiert enthalten, finden Verwendung in der Wasseraufbereitung, beispielsweise als Ionentauscherharze oder als Bestandteil von Membranen.

In dieser Schrift verwendete ppm- und Prozentangaben beziehen sich, falls nicht anders angegeben, auf Gewichtsprozente und - ppm.

Beispiel 1

Einer Umesterungsapparatur, die aus einem Umesterungsreaktor (Füllvolumen 1,8 1) mit außenliegendem Umlaufverdampfer und aufgesetzter Destillationskolonne (20 Dual-Flow-Böden) mit Kondensator und einem Nachreaktor (Füllvolumen 1,2 1) mit außenliegendem Umlaufverdampfer bestand, wurden im kontinuierlichen Betrieb stündlich 134 g Dimethyl-aminoethanol, 175 g n-Butylacrylat (Reinheit 99,6%, 0,1 % Dibutylether, 0,05 % Butylacetat, 0,03 % Butylpropionat, 0,04 % Wasser, 0,1 % Isobutylacrylat, 0,01 % Acrylsäure, 0,02 % Pentylacrylate), 6 g Titantetrabutylat, 71 g Rückkatalysator und 833 g Rückstrom aus der Reindestillation, der im wesentlichen aus n-Butylacrylat (ca. 75 %) und Dimethylaminoethanol (ca. 17 %) bestand, zugeführt. Die Temperatur im Reaktor betrug 120°C, im Nachreaktor 123°C. Über den Kopf der Kolonne wurde eine n-Butanolfraktion abgetrennt, die hauptsächlich aus Butanol (83%) und Butylacrylat (17 %) bestand, und kondensiert. 132 g des Kondensats wurden ausgeschleust, der Rest (ca. 1400 g) wurde als Rücklauf auf den obersten Kolonnenboden aufgebracht. Auf den Kopf des Kondensators wurden stündlich 15 g einer Lösung von 0,5 % Phenothiazin in n-Butylacrylat aufgesprüht. In die Umlaufverdampfer wurden jeweils 10 1 Luft/h eingeblasen. Die Verweilzeit betrug 2,6 h.

Der Austrag des Nachreaktors wurde einer Destillationseinheit zugeführt, die aus einer Destillationskolonne (8 Dual-Flow-Böden) mit Umlaufverdampfer und Kondensator bestand (Sumpftemperatur 105 °C), und die Schwersieder (85 g/h), hauptsächlich aus Zielester (ca. 65 %), Tetra-dimethylaminoethyltitanat (ca. 30 %) und n-Butylacrylat (ca. 3 %) bestehend, abgetrennt, wobei ca. 80 % in den Umesterungsreaktor zurückgeführt wurden und der Rest (17 g/h) ausgeschleust wurde. Das am Kopf der Kolonne (69°C / 50mbar) anfallende Produktgemisch (ca. 60 % n-Butylacrylat, ca. 13 % Dimethylaminoethanol, ca. 25 % Zielester, ca. 2,5 % n-Butanol) wurde kondensiert und teilweise (50 %) als Rücklauf wieder auf den Kolonnenkopf aufgebracht. Zwecks Stabilisierung wurde eine Lösung (15 g/h) von 0,5 % Phenothiazin in n-Butylacrylat in den Kondensator eingesprüht. Der Rest des Destillats (ca. 1030 g/h) wurde einer weiteren Destillationseinheit, die aus einer Kolonne mit 22 Dual-Flow Böden mit Seitenabzug, Kondensator und Umlaufverdampfer bestand, zugeführt. Am Kopf der Kolonne wurde ein Gemisch aus ca. 16,7 % Dimethylaminoethanol, ca. 75 % n-Butylacrylat, ca. 5 % Zielester und ca. 3 % n-Butanol abgetrennt, teilweise (ca. 40 %) als Rücklauf wieder am Kopf der Kolonne aufgebracht und teilweise (833 g/h) in den Umesterungsreaktor zurückgeführt. Die Stabilisierung erfolgte mit einer Lösung von 0,5 % Phenothiazin und 0,05 % 4-Hydroxy-2,2,6,6-tetramethylpiperidin-N-Oxyl. Das Sumpfprodukt (ca. 10 g/h), hauptsächlich Zielester (ca. 99%), wurde der Katalysatorabtrennung zugeführt. Das über den

Seitenabzug gasförmig abgetrennte Dimethylaminoethylacrylat wurde kondensiert (206 g/h) und mit ca. 20 ppm Hydrochinonmonomethylether stabilisiert.

In die Umlaufverdampfer der beiden Destillationseinheiten wurde jeweils 10 l/h Luft eingeleitet.

Die Ausbeute an destilliertem Produkt betrug 96 % (bezüglich Dimethylaminoethanol). Die gaschromatographisch ermittelte Reinheit betrug 99,9 %, wobei als Nebenkomponenten < 20 ppm Dimethylaminoethanol, ca. 100 ppm Butylacrylat, ca. 250 ppm Wasser, ca. 300 ppm Dimethylaminoethylpropionat und < 10 ppm Dibutylether gefunden wurden.

N,N'-Dimethylpiperazin, Ethylenglykoldi(meth)acrylat und Vinyloxyethyl(meth)acrylat konnten nicht nachgewiesen werden.

Die Anlage wurde ohne Polymerisatprobleme 30 Tage betrieben.

Beispiel 2 (als Vergleich)

Es wurde wie in Beispiel 1 aber ohne Spülgas in den Umlaufverdampfern und Wärmetauschern verfahren.

Innerhalb von 5 Tagen stieg im Zielester der Gehalt an Dimethylpiperazin auf ca. 100 ppm und an Vinyloxyethylacrylat auf ca. 20 ppm. Nach 7 Tagen mußte die Apparatur wegen Belegung der Wärmetauscherrohre mit Polymerisat abgestellt werden.

Beispiel 3

Es wurde der Einfluß der Verweilzeit auf die Bildung von Nebenprodukten (Michael-Additionsprodukte und N, N'-Dimethylpiperazin) im Reaktoraustrag untersucht.

Es wurde gemäß dem im Beispiel 1 beschriebenen Umesterungsverfahren vorgegangen. Der Austrag wurde gaschromatographisch nach Abtrennung des Katalysators durch Hydrolyse und Filtration analysiert.

| Verweilzeit | Michael-Additionsprodukt | N,N'-Dimethyl-piperazin |
|---|---|---|
| 2 Stunden | 2,5 % | 0,8 % |
| 3 Stunden | 4,6 % | 1,5 % |
| 5 Stunden | 6,0 % | 2,5 % |
| 7 Stunden | 6,9 % | 3,1 % |

Beispiel 4

Es wurde analog Beispiel 1 verfahren, jedoch wurde die Verweilzeit auf 5 Stunden eingestellt.

Man erhielt eine Ausbeute von 93 % bezüglich Dimethylaminoethanol, der Gehalt an N,N'-Dimethylpiperazin im Zielester betrug 60 ppm.

Vergleichsbeispiel 1

Im kontinuierlichen Betrieb wurden stündlich 680 Teile Dimethylaminoethanol, 2016 Teile eines Gemisches aus Ethylacrylat und dem Destillat der Ethylacrylat-Abtrennung, 70 Teile Katalysatorlösung aus Beispiel 1 und 110 Teile Rückkatalysator (Sumpf der Katalysatorabtrennung) dem ersten Reaktor einer aus zwei Reaktoren bestehenden Reaktorkaskade zugeführt. Die Reaktoren waren jeweils mit einer aufgesetzten Füllkörperkolonne und einem Kondensator ausgerüstet. Die Wärmezufuhr erfolgte über außenliegende Wärmetauscher. Außerdem wurden 236 Teile des Kondensats der Leichtsiederabtrennung stündlich über einen Zulauf in der Mitte der Kolonne des ersten Reaktors zugegeben. Der Austrag des zweiten Reaktors wurde einem mit einem Umlaufverdampfer ausgerüsteten Behälter zugeführt, der gasseitig mit der Kolonne des zweiten Reaktors verbunden war. Die Reaktionstemperaturen betrugen 110 beziehungsweise 115 ° C, im Behälter 119 °C. Das bei der Umesterung entstehende Ethanol wurde als Gemisch mit Ethylacrylat (48 % Ethanol) am Kopf der Reaktorkolonnen ausgeschleust und kondensiert. Die vereinigten Kondensate wurden teilweise als Rücklauf in die Kolonnen zurückgeführt (jeweils ca. 2100 Teile) und der Rest (806 Teile) ausgeschleust und für eine Verwendung in der Ethylacrylatherstellung gesammelt. Die Stabilisierung der Kondensate erfolgt durch Zugabe von 120 Teilen einer Lösung von 0,5 % Phenothiazin und 0,05 % 4-Hydroxy-2,2,6,6-tetramethylpiperidin-N-Oxyl in Ethylacrylat auf jeden Kondensator. Die vereinigten Kondensate enthielten ca. 48 Gew.% Ethanol und ca. 52 Gew.% Ethylacrylat, der Gehalt an Dimethylaminoethanol betrug weniger als 0,1 %.

Der Austrag des Behälters wurde einer Füllkörperkolonne zugeführt (Zulauf in der Kolonnenmitte), wo er in ein

Kopfprodukt (Kopftemperatur 88° C, 500 mbar) und ein katalysatorhaltiges Sumpfprodukt (Sumpftemperatur 140 °C) aufgetrennt wurde.

Das Kopfprodukt wurde durch Einsprühen von 110 Teilen der oben beschriebenen Stabilisatorlösung in den Kondensatorkopf stabilisiert. Das Kondensat wurde mit 1170 Teilen Ethylacrylat vermischt und teilweise (380 Teile/h) als Rücklauf wieder der Kolonne und der Rest (2016 Teile/h) dem ersten Reaktor zugeführt.

Das Sumpfprodukt wurde einem Flashverdampfer zugeführt, der zusätzlich mit einem Umlaufverdampfer ausgerüstet war (135 °C, 80 mbar). Das Destillat wurde mit 50 Teilen Stabilisatorlösung (s.o.) stabilisiert und enthielt ca. 90 % Dimethylaminoethylacrylat. Das Sumpfprodukt wurde teilweise (110 Teile) dem ersten Reaktor zugeführt und der Rest (70 Teile/h) der Rückstandsbehandlung zugeführt.

Das Verhältnis der Michael-Additionsproduktbildung (EP-A 906 902, Seite 9) war, über beide Destillationsstufen berechnet, negativ (-1,3%), das bedeutet eine teilweise Reduzierung (Rückspaltung) der Addukte unter den angegebenen Bedingungen.

Die weitere, analog Beispiel 2 durchgeführte Aufarbeitung des Destillats der Flashverdampfung lieferte 1189 Teile Dimethylaminoethylacrylat in einer Reinheit von 99,9 %, was einer Ausbeute von 97,9 % bezüglich Dimethylaminoethanol entspricht.

Vergleichsbeispiel 2

Es wurde analog Vergleichsbeispiel 1 verfahren, die Sumpftemperatur bei der Ethylacrylatabtrennung betrug jedoch 110 °C und die Temperatur bei der Katalysatorabtrennung (Flashverdampfer) 110 °C.

Das auf Basis der gaschromatographischen Analysen der einzelnen Ströme ermittelte Verhältnis der Michael-Additionsproduktbildung betrug +0,1%, d.h. es erfolgte eine geringe Zunahme an Michael-Additionsprodukt.

## Patentansprüche

1. Verfahren zur Herstellung von basischen (Meth)acrylsäureestern IV durch Umesterung von (Meth)acrylsäurealkylestern I

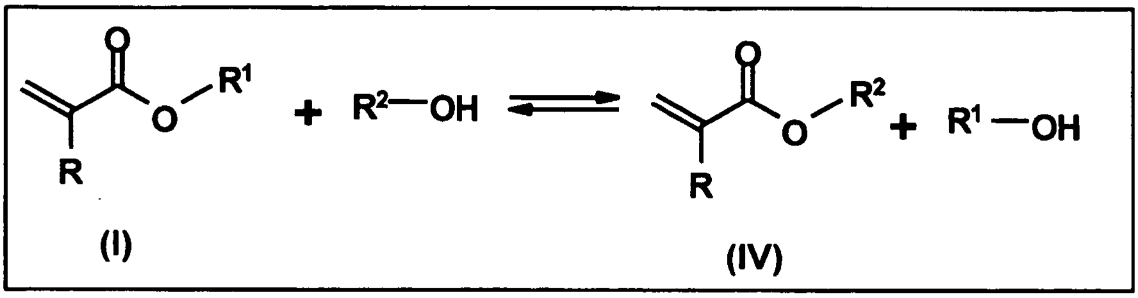

worin

R Wasserstoff oder Methyl,
$R^1$ ein Alkylrest eines ein bis sechs Kohlenstoffatome auf- weisenden Alkohols,
$R^2$ ein zwei bis zwölf Kohlenstoffatome umfassender, gerad- kettiger oder verzweigter, gesättigter oder ungesättig- ter Alkylrest, substituiert mit mindestens einer $NR^3_2$ - Gruppe
$R^3$ ein zwei bis sechs Kohlenstoffatome umfassender, gerad- kettiger oder verzweigter, gesättigter oder ungesättig- ter Alkylrest, wobei N mit den Substituenten $R^3$ auch ei- nen fünf- bis siebengliedrigen Ring bilden kann und die Substituenten $R^3$ gleich oder verschieden sein können,

bedeuten,
in Gegenwart eines Katalysators und destillativer Aufarbeitung des Reaktionsgemisches, **dadurch gekennzeichnet, daß** man ein unter den Reaktionsbedingungen inertes Gas oder Gasgemisch durch die Reaktionszone und/ oder Wärmetauscher leitet.

2. Verfahren nach Anspruch 1, **dadurch gekennzeichnet, daß** es sich bei dem inerten Gas oder Gasgemisch um Luft oder Luft-Stickstoffgemische handelt.

3. Verfahren nach Anspruch 1 oder 2, **dadurch gekennzeichnet, daß** die Reaktionszone einen Nachreaktor beinhaltet.

4. Verfahren nach einem der Ansprüche 1 bis 3, **dadurch gekennzeichnet, daß** die Verweilzeit des Reaktionsgemisches in der Reaktionszone 1,5 bis 3 Stunden beträgt.

5. Verfahren nach einem der Ansprüche 1 bis 4, **dadurch gekennzeichnet, daß** man die Umesterung in einer Reaktionszone mit mindestens einer aufgesetzten Kolonne durchführt und am Kopf der Kolonne ein nichtazeotropes Gemisch abnimmt, bei dem gegenüber der azeotropen Zusammensetzung aus niederem Alkanol $R^1OH$ und niederem (Meth)acrylsäureester I bei den entsprechenden Bedingungen der Gehalt an niederem (Meth) acrylsäureester I erhöht ist.

6. Verfahren nach einem der Ansprüche 1 bis 5, **dadurch gekennzeichnet, daß** der basische (Meth)acrylsäureester IV in der Reindestillation gasförmig über einen Seitenabzug abgenommen wird.

7. Verfahren nach einem der Ansprüche 1 bis 6, **dadurch gekennzeichnet, daß** die Umesterung in Gegenwart eines Titantetraalkoholats durchgeführt wird, wobei das Alkoholat der Alkoholkomponente $R^1OH$ des niederen (Meth) acrylsäurealkylesters I entspricht.

8. Verfahren nach einem der Ansprüche 1 bis 7, **dadurch gekennzeichnet, daß** die Stabilisierung des Reaktionsgemisches während der Aufarbeitung mit Phenothiazin oder einem Gemisch aus Phenothiazin und mindestens einem weiteren Stabilisator durchgeführt wird.

9. Verfahren nach einem der Ansprüche 1 bis 8, **dadurch gekennzeichnet, daß** die Stabilisierung des Reaktionsgemisches während der Aufarbeitung mit einem Gemisch aus Phenothiazin und 4-Hydroxy-2,2,6,6-tetramethylpiperidin-N-Oxyl durchgeführt wird.

10. Verfahren nach einem der Ansprüche 1 bis 9, **dadurch gekennzeichnet, daß** zumindest ein Teil der bei der Aufarbeitung anfallenden Destillationssümpfe einer Katalysatorabtrennung zugeführt wird.

11. Verfahren nach einem der Ansprüche 1 bis 10, **dadurch gekennzeichnet, daß** der für die Umesterung eingesetzte niedere (Meth)acrylsäureester I ausgewählt ist aus (Meth)acrylsäuremethylester, (Meth)acrylsäureethylester, (Meth) acrylsäure-n-Propylester, (Meth)acrylsäure-iso-propylester, (Meth)acrylsäure-n-butylester, (Meth)acrylsäure-iso-butylester, (Meth)acrylsäure-sec-butylester, (Meth)acrylsäure-tert.-butylester, (Meth)acrylsäure-n-pentylester und (Meth)acrylsäure-n-hexylester.

12. Verfahren nach Anspruch 11, **dadurch gekennzeichnet, daß** es sich bei dem für die Umesterung eingesetzten niederen (Meth)acrylsäurealkylester I um (Meth)acrylsäure-n-butylester handelt.

13. Verfahren nach Anspruch 11 oder 12, **dadurch gekennzeichnet, daß** der (Meth)acrylsäure-n-butylester in technischer Reinheit eingesetzt wird.

14. Verfahren nach einem der Ansprüche 1 bis 13, **dadurch gekennzeichnet, daß** der basische (Meth)acrylsäureester IV ausgewählt ist aus 2-Dimethylaminoethyl(meth)acrylat, 3-Dimethylaminopropyl(meth)acrylat, 1-Dimethylaminoprop-2-yl(meth)acrylat, 2-Dimethylaminopropyl(meth)acrylat, 6-Dimethylaminohexyl(meth)acrylat, 2-Diethylaminoethyl(meth)acrylat, 3-Diethylaminopropyl(meth)acrylat, 6-Diethylaminohexyl(meth)acrylat, 2-Dibutylaminoethyl (meth)acrylat, 3-Dibutylaminopropyl(meth)acrylat und 6-Dibutylaminohexyl(meth)acrylat.

15. Verfahren nach Anspruch 14, **dadurch gekennzeichnet, daß** es sich bei dem basischen (Meth)acrylsäureester IV um Dimethylaminoethylacrylat handelt.

## Claims

1. A process for the preparation of basic (meth)acrylates IV by transesterification of alkyl (meth)acrylates I

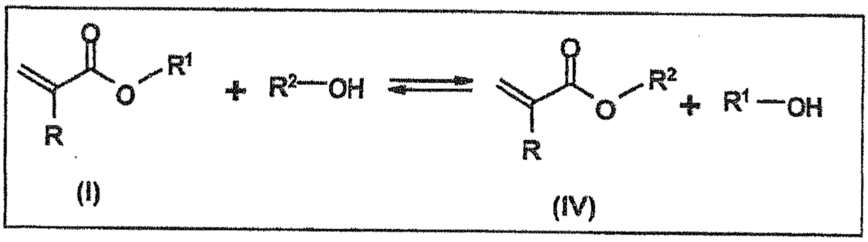

in which

R is hydrogen or methyl,
$R^1$ is an alkyl radical of an alcohol having one to six carbon atoms,
$R^2$ is a straight-chain or branched, saturated or unsaturated alkyl radical comprising two to twelve carbon atoms and substituted by at least one $NR^3_2$ group,
$R^3$ is a straight-chain or branched, saturated or unsaturated alkyl radical comprising two to six carbon atoms, it being possible for N with the substituents $R^3$ to also form a five- to seven- membered ring and for the substituents $R^3$ to be identical or different,

in the presence of a catalyst and working-up of the reaction mixture by distillation, wherein a gas or gas mixture which is inert under the reaction conditions is passed through the reaction zone and/or heat exchanger.

**2.** A process according to claim 1, wherein the inert gas or gas mixture is air or an air/nitrogen mixture.

**3.** A process according to claim 1 or 2, wherein the reaction is zone comprises a postreactor.

**4.** A process according to any of claims 1 to 3, wherein the residence time of the reaction mixture in the reaction zone is from 1.5 to 3 hours.

**5.** A process according to any of claims 1 to 4, wherein the transesterification is carried out in a reaction zone having at least one attached column and a nonazeotropic mixture is taken off at the top of the column, in which mixture the content of lower (meth)acrylate I is higher compared with the azeotropic composition comprising lower alkanol $R^1OH$ and lower (meth)acrylate I under the corresponding conditions.

**6.** A process according to any of claims 1 to 5, wherein the basic (meth)acrylate IV is taken off in the purification by distillation in gaseous form via a side take-off.

**7.** A process according to any of claims 1 to 6, wherein the transesterification is carried out in the presence of a titanium tetraalcoholate, the alcoholate corresponding to the alcohol component R10H of the lower alkyl (meth)acrylate I.

**8.** A process according to any of claims 1 to 7, wherein the stabilization of the reaction mixture during the working-up is carried out using phenothiazine or a mixture of phenothiazine and at least one further stabilizer.

**9.** A process according to any of claims 1 to 8, wherein the stabilization of the reaction mixture during the working-up is carried out using a mixture of phenothiazine and 4-hydroxy-2,2,6,6-tetramethylpiperidin-N-oxyl.

**10.** A process according to any of claims I to 9, wherein at least a part of the distillation bottom products obtained during the working-up is subjected to a catalyst removal.

**11.** A process according to any of claims 1 to 10, wherein the lower (meth)acrylate I used for the transesterification is selected from methyl (meth)acrylate, ethyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-pentyl (meth)acrylate and n-hexyl (meth)acrylate.

**12.** A process according to claim 11, wherein the lower alkyl (meth)acrylate I used for the transesterification is n-butyl (meth)acrylate.

**13.** A process according to claim 11 or 12, wherein the n-butyl (meth)acrylate is used in industrial purity.

**14.** A process according to any of claims 1 to 13, wherein the basic (meth)acrylate IV is selected from 2-dimethylaminoethyl (meth)acrylate, 3-dimethylaminopropyl (meth)acrylate, 1-dimethylaminoprop-2-yl (meth)acrylate, 2-dimethylaminopropyl (meth)acrylate, 6-dimethylaminohexyl (meth)acrylate, 2-diethylaminoethyl (meth)acrylate, 3-diethylaminopropyl (meth)acrylate, 6-diethylaminohexyl (meth)acrylate, 2-dibutylaminoethyl (meth)acrylate, 3-dibutylaminopropyl (meth)acrylate and 6-dibutylaminohexyl (meth)acrylate.

**15.** A process according to claim 14, wherein the basic (meth)acrylate IV is dimethylaminoethyl acrylate.

## Revendications

**1.** Procédé de fabrication d'esters de l'acide (méth)acrylique basiques IV par transestérification d'esters alkyliques de l'acide (méth)acrylique I

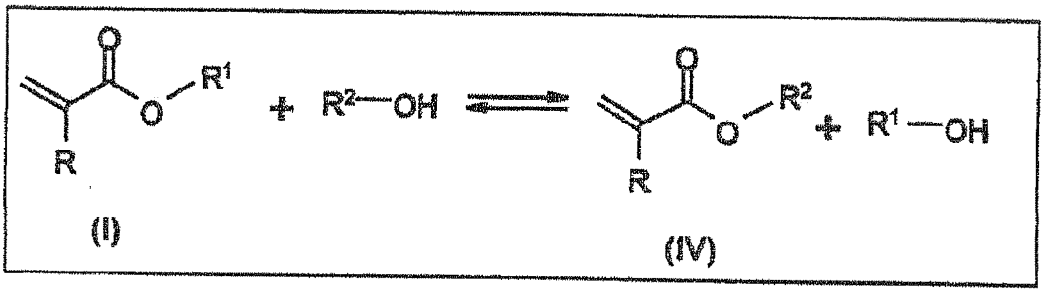

avec les significations suivantes :

R hydrogène ou méthyle,
$R^1$ un radical alkyle d'un alcool comprenant un à six atomes de carbone,
$R^2$ un radical alkyle comprenant deux à douze atomes de carbone, linéaire ou ramifié, saturé ou insaturé, substitué avec au moins un groupe $NR^3_2$,
$R^3$ un radical alkyle comprenant deux à six atomes de carbone, linéaire ou ramifié, saturé ou insaturé, N pouvant également former un cycle de cinq à sept éléments avec les substituants $R^3$ et les substituants $R^3$ pouvant être identiques ou différents,

en présence d'un catalyseur et traitement distillatif du mélange réactionnel, **caractérisé en ce qu'**un gaz ou mélange de gaz inerte dans les conditions de la
réaction est passé dans la zone de réaction et/ou l'échangeur de chaleur.

**2.** Procédé selon la revendication 1, **caractérisé en ce que** le gaz ou mélange de gaz inerte est l'air ou des mélanges air-azote.

**3.** Procédé selon la revendication 1 ou 2, **caractérisé en ce que** la zone de réaction contient un réacteur secondaire.

**4.** Procédé selon l'une quelconque des revendications 1 à 3, **caractérisé en ce que** le temps de séjour du mélange réactionnel dans la zone de réaction est de 1,5 à 3 heures.

**5.** Procédé selon l'une quelconque des revendications 1 à 4, **caractérisé en ce que** la transestérification est réalisée dans une zone de réaction sur laquelle est placée au moins une colonne et un mélange non azéotropique est soutiré à la tête de la colonne, dont la teneur en ester de l'acide (méth)acrylique inférieur I est élevée par rapport à la composition azéotropique de l'alcanol inférieur $R^1OH$ et de l'ester de l'acide (méth)acrylique inférieur I dans les conditions correspondantes.

**6.** Procédé selon l'une quelconque des revendications 1 à 5, **caractérisé en ce que** l'ester de l'acide (méth)acrylique basique IV est soutiré sous forme gazeuse par une sortie latérale lors de la purification par distillation.

7. Procédé selon l'une quelconque des revendications 1 à 6, **caractérisé en ce que** la transestérification est réalisée en présence d'un tétraalcoolate de titane, l'alcoolate correspondant au composant alcool $R^1OH$ de l'ester alkylique de l'acide (méth)acrylique inférieur I.

8. Procédé selon l'une quelconque des revendications 1 à 7, **caractérisé en ce que** la stabilisation du mélange réactionnel pendant le traitement est réalisée avec de la phénothiazine ou un mélange de phénothiazine et d'au moins un stabilisateur supplémentaire.

9. Procédé selon l'une quelconque des revendications 1 à 8, **caractérisé en ce que** la stabilisation du mélange réactionnel pendant le traitement est réalisée avec un mélange de phénothiazine et de 4-hydroxy-2,2,6,6-tétraméthylpipéridine-N-oxyle.

10. Procédé selon l'une quelconque des revendications 1 à 9, **caractérisé en ce qu'**au moins une partie des fonds de distillation formés lors du traitement est introduite dans une séparation du catalyseur.

11. Procédé selon l'une quelconque des revendications 1 à 10, **caractérisé en ce que** l'ester de l'acide (méth)acrylique inférieur I utilisé pour la transestérification est choisi parmi l'ester méthylique de l'acide (méth)acrylique, l'ester éthylique de l'acide (méth)acrylique, l'ester n-propylique de l'acide (méth)acrylique, l'ester iso-propylique de l'acide (méth)acrylique, l'ester n-butylique de l'acide (méth)acrylique, l'ester iso-butylique de l'acide (méth)acrylique, l'ester sec-butylique de l'acide (méth)acrylique, l'ester tert.-butylique de l'acide (méth)acrylique, l'ester n-pentylique de l'acide (méth)acrylique et l'ester n-hexylique de l'acide (méth)acrylique.

12. Procédé selon la revendication 11, **caractérisé en ce que** l'ester alkylique de l'acide (méth)acrylique inférieur I utilisé pour la transestérification est l'ester n-butylique de l'acide (méth)acrylique.

13. Procédé selon la revendication 11 ou 12, **caractérisé en ce que** l'ester n-butylique de l'acide (méth)acrylique est utilisé en une pureté technique.

14. Procédé selon l'une quelconque des revendications 1 à 13, **caractérisé en ce que** l'ester de l'acide (méth)acrylique basique IV est choisi parmi le (méth)acrylate de 2-diméthylaminopropyle, le (méth)acrylate de 3-diméthylaminopropyle, le (méth)acrylate de 1-diméthylaminoprop-2-yle, le (méth)acrylate de 2-diméthylaminopropyle, le (méth)acrylate de 6-diméthylaminohexyle, le (méth)acrylate de 2-diéthylaminoéthyle, le (méth)acrylate de 3-diéthylaminopropyle, le (méth)acrylate de 6-diéthylaminohexyle, le (méth)acrylate de 2-dibutylaminoéthyle, le (méth)acrylate de 3-dibutylaminopropyle et le (méth)acrylate de 6-dibutylaminohexyle.

15. Procédé selon la revendication 14, **caractérisé en ce que** l'ester de l'acide (méth)acrylique basique IV est l'acrylate de diméthylaminoéthyle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE AS1248943 B
- EP 906902 A2
- EP 906902 A
- EP 960877 A
- FR 2617840
- US 2832800 A
- EP 298867 B1
- EP 960877 A2
- DE OS2008618 A
- DE PS1067806 C
- EP 160427 A
- DE OS2805702 A
- JP 3112949 A
- DE 10127941


### In der Beschreibung aufgeführte Nicht-Patentliteratur


- Organikum. VEB Deutscher Verlag der Wissenschaften, 1988